# EUROPEAN PATENT APPLICATION

(11) **EP 1 555 312 A1**
(43) Date of publication of application: **20.07.2005**
(21) Application number: 04445126.8
(22) Date of filing: 13.12.2004
(51) Int. Cl.: C12M 1/02, C12M 1/04, C12M 1/16

(54) **Method and means for multiplying microorganisms**

(30) Priority: 16.12.2003 SE 0303435
(71) Applicant: Nilsson, Kurt, 186 92 Vallentuna (SE); Nilsson, Martin, 186 55 Vallentuna (SE)
(72) Inventor: Nilsson, Kurt, 186 92 Vallentuna (SE); Nilsson, Martin, 186 55 Vallentuna (SE)
(74) Representative: Wiedemann, Bernd

(57) **Abstract**

The invention relates to a method of producing a slurry of microorganisms in an aqueous medium, wherein the slurry contains a very large quantity of microorganisms as a result of creating an environment that promotes a significant increase/growth of the microorganisms slurried in the aqueous medium. The invention also relates to an arrangement (108) for carrying out the method. The slurry is intended for use in decontaminating primarily hydrocarbon contaminated areas of the ground/soil and contaminated water present in said areas, such water consisting at least partially of groundwater in many instances.

The slurry of microorganisms is produced by delivering freeze-dried microorganisms to a cavity (204) that is surrounded by porous material (203). The pores of the porous material and the cavity located therein are filled with an aqueous medium that contains nutrients, trace elements and other substances necessary for the growth of the microorganisms. Mixing or stirring of the slurry is achieved with the aid of an oxygen containing gas, normally air, delivered to the lower part of the porous material. The gas rises upwards and is therewith dissolved partially in the medium and results in mixing of the medium. The pores in the porous material are mutually connected through passageways and provide beneficial conditions for the growth of said microorganisms.

## Description

The present invention relates to a method of producing a slurry of microorganisms in an aqueous medium, said slurry being caused to contain a very large number of microorganisms, or copies thereof, by creating an environment that promotes a significant increase/growth of microorganisms slurried in the aqueous medium, and also to an arrangement for carrying out the method. The slurry containing said copious quantity of microorganisms is intended for use in the decontamination of primarily hydrocarbon-contaminated ground areas and the water present therein, such water consisting in many instances at least partially of groundwater.

It is known to use different microorganisms for the destruction of, *inter alia,* hydrocarbons, for instance such organisms as Arthrobacter sp., Bacillus megaterium, B. subtilis and B. thurengiensis. The residual product is carbon dioxide and water. Such microorganisms can also be used to degrade other organic compounds, such as fats and halogenated hydrocarbons, for instance PCB and vinyl chloride.

Hydrocarbon contaminated ground/soil/earth and areas of water can be decontaminated either in-situ or ex-situ. According to the present invention, the resultant slurry can be used for decontamination purposes in all of the aforesaid instances, although with the application of methods of a varying nature. In the latter instances, the contaminated soil is excavated and treated in a separate plant and then returned to the ground after decontamination. When treating in-situ, the soil/earth or the water-carrying ground layer is treated with slurry that has a high microorganism content in the aqueous medium, said slurry being administered to the contaminated ground layer through the medium of infiltration pipes. The length of these pipes is, in principle, equal to the depth of the contaminated ground layer.

Spaced from the infiltration pipes are pumping pits in which groundwater and infiltrated water containing microorganisms is collected and pumped to a container. The major part of the microorganisms delivered to the contaminated ground fasten in pores of the soil, therewith depleting the water from the infiltration pipes of microorganisms at progressively increasing distances from said pipes. Other microorganisms float in the water-carrying layer and therewith degrade the hydrocarbons present in both the soil and in the water.

The water obtained from the pumping pits contains both microorganisms and hydrocarbons extracted from the ground (earth or soil). This water is treated in different ways.

When decontamination shall be effected solely in water masses, the water present is treated in a separate plant and then returned. In the case of water sanitation, the water is supplied with a slurry that has a high microorganism content, for decontamination of hydrocarbon compounds, among others.

The decontamination of soil and water-containing areas contaminated by hydrocarbons is a time-consuming exercise. The time taken to sanitise hydrocarbon contaminated soil areas will depend, *inter alia,* on the degree of contamination, the volume of soil that is contaminated and/or the volume of the contaminated water area to be treated, and also the amount of microorganisms delivered to said areas. Of these factors influential with regard to the time taken to achieve decontamination, it is the number of microorganisms delivered to said areas that can be influenced.

Hitherto, it has been possible to produce microorganism slurries that contain up to 10⁷ microorganisms per ml of water. This quantity is insufficient, however, to achieve ground sanitation with a satisfactory result over an acceptable sanitation time period.

One object of the present invention is to produce in an aqueous medium a microorganism slurry for the sanitation of soil and areas of water contaminated with hydrocarbons, said aqueous medium containing a significantly greater amount of slurried microorganisms than what has hitherto been achievable.

Another object of the invention is to provide an arrangement for the production of a microorganism slurry that is obtained in accordance with the present method.

The first object of the invention is achieved with a method of producing a microorganism slurry in a liquid medium consisting essentially of water, wherein the method is characterised by introducing freeze-dried microorganisms into a cavity provided in a material that has mutually communicating pores, said material being located in a lower part of a container, by delivering to the container an aqueous medium, nutrients and trace elements for promoting the copious growth of the microorganisms, and by introducing an oxygen containing gas into the lower part of the container beneath said material, such that the gas will rise up through said porous material. The oxygen-containing gas is preferably air, although it may also consist of oxygen enriched air or of pure oxygen.

According to one preferred embodiment of the invention, the aqueous medium used is water that contains said hydrocarbons. This may consist of water deriving from pumping pits in contaminated ground areas. Such water will also contain microorganisms and other constituents dissolved, emulsified or slurried in the water.

According to another embodiment, there is used water from pumping pits that has been collected in a receiving tank.

Freeze-dried microorganisms are delivered to the cavity in the porous material, in order to obtain quickly a high initial content of microorganisms in the container. The porous material will preferably have a porosity of 16-140 pores per cm², (64-1650 pores per cm³), preferably within the range of 40-100 pores per cm², (250-1000 pores per cm³). The aqueous medium present in the container will optimally be kept at a temperature within the range of 20-35 degrees Celsius.

The pH-value of the aqueous medium will preferably lie within the range of 5-10, a preferred pH-value lying in the range of 6-8. Adjustments are made as required with respect to environmentally friendly acids or bases, for instance carbon dioxide and sodium hydrocarbonate or potassium hydrocarbonate.

More than 5*10⁸ microorganisms per ml water have been obtained with the aid of the inventive method.

An arrangement for greatly increasing the number of microorganisms in accordance with the inventive method comprises a container that has an inlet opening for the supply of aqueous medium that includes nutrients and trace substances and, for instance, pH-controlling additives for promoting the growth of microorganisms, and an outlet opening through which the slurry is carried away, a layer of porous material that has mutually communicating pores and includes a cavity which is surrounded by the porous material, said cavity being located in the lower part of the container and intended to accommodate freeze-dried microorganisms, wherein the arrangement also includes means for delivering oxygen-containing gas to the lower part of the porous material, preferably to the container at a location beneath the layer of porous material, and a gas outlet opening in the upper part of the container.

The layer of porous material has a thickness of 20-70 cm, preferably a thickness of 30-50 cm. The porous material has a porosity of 16-140 pores per cm² (64-1650 pores per cm³), preferably a porosity in the range of 40-100 pores per cm², (250-1000 pores per cm³). The material may conveniently consist of a cellular urethane plastic.

It is preferred that the container is an openable container and that a supply line extends through the container wall and into the cavity in the porous material for delivering freeze-dried microorganisms to the cavity. The porous material will preferably take-up at most 50 percent of the height of the container and will preferably have roughly the same diameter or cross-dimension as the container interior.

The invention will now be described in more detail with reference to the accompanying drawing, in which
Figure 1 is a diagrammatic illustration of a system for the in-situ decontamination of a ground area;
Figure 2 is a diagrammatic illustration of a bioreactor for batch-wise manifolding of microorganisms;
Figure 3 illustrates a bioreactor similar to that shown in Figure 2, for the continuous manifolding of microorganisms; and
Figure 4 is a graph illustrating the reduction of hydrocarbons as a function of time.

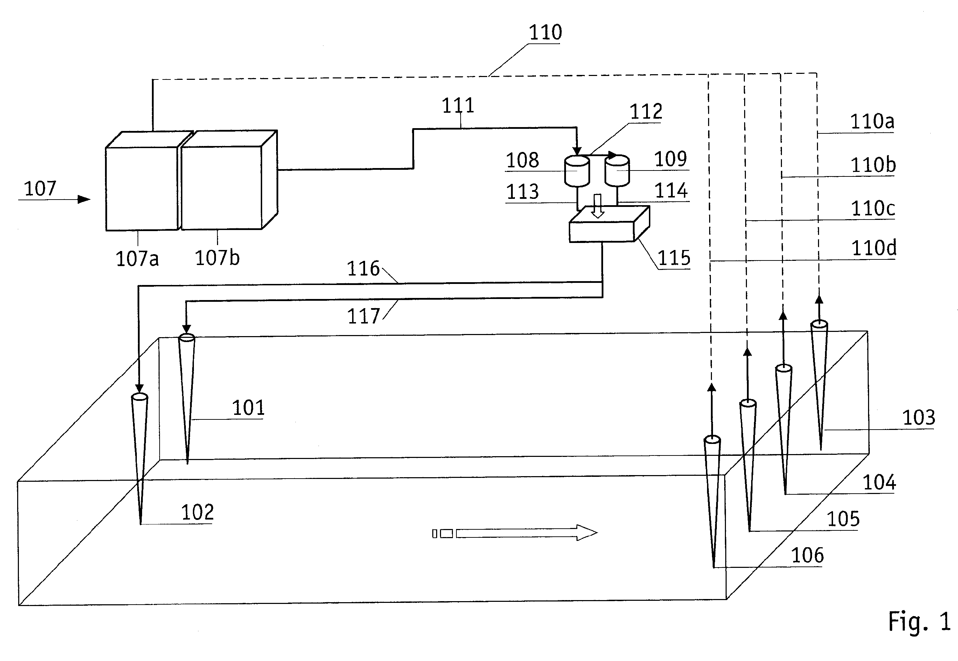

The system illustrated in Figure 1 comprises two infiltration pipes 101, 102 that are sunk into a contaminated ground area. Spaced from the infiltration pipes 101, 102 are four pumping pits 103-106. Extending from each of the pumping pits 103-106 is a respective line 110a, 110b, 110c and 110d, said lines being joined in a common line 110. The line 110 opens into a water processing unit 107 that includes a settling tank 107a and a micro-organism cultivation tank 107b. The settling tank 107a and the cultivation tank 107b are mutually connected. Devices used for the delivery of water, nutrients, trace substances and oxygen-containing gas, such as air, have not been shown in the figure.

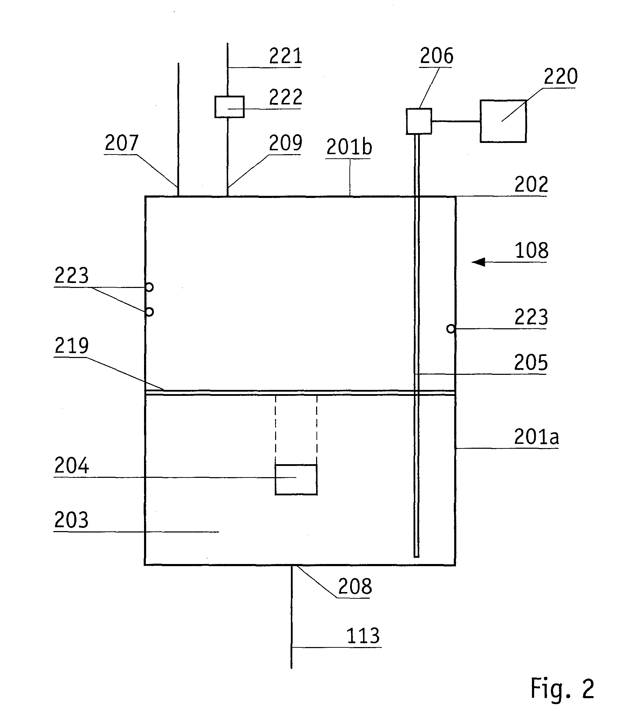

Also included in the system are two organism accelerators 108, 109, which are driven batch-wise and connected for alternate operation. These organism accelerators 108, 109 are mutually identical units and will be described below with reference to Figure 2. The accelerators are connected to the cultivation tank 107b by a line 111 which opens into the first accelerator 108. A branch line 112 extends from the line 111 to the second accelerator 109. Although not shown, valves for controlling and regulating pressure and resistance are mounted in the lines 111 and 112 downstream of the branch.

The accelerators 108 and 109 are also each connected to respective lines 113 and 114 which open into a collecting tank 115. The collecting tank 115 is connected to the infiltration pipes 101, 102 via respective lines 116 and 117.

Aqueous medium that has an extremely high content of microorganisms is pumped from the collecting tank 115 (by pumps not shown) to the infiltration pipes 101, 102. The medium is then spread from the pipes throughout the ground area, preferably in the direction of the arrows P. As it passes through the ground area, most of the microorganisms will fasten in the ground cavities and degrade the hydrocarbons present in the ground or soil. Aqueous slurry, which may contain soil or earth particles, that reaches the pumping pits 103-106 is pumped through the lines 110a-110d to the water processing unit 107. Those particles that settle onto the bottom of the sedimentation tank 107a are separated from the water. The sediment and part of the water freed from particles are passed to a separate processing or treatment station. The remainder of the water, which also contains microorganisms, is passed to the cultivation tank 107b.

Oxygen-containing gas, nutrients, trace substances and possibly also other substances required for the growth of microorganisms are added to the cultivation tank 107b. The water containing said microorganisms is then passed to one of the organism accelerators 108 or 109. Freeze-dried microorganisms, water from the cultivating tank 107b and other constituents necessary for the growth of microorganisms are added to said accelerator. The water thus enriched in microorganisms is then led to the collecting tank and from there to the infiltration pipes.

The organism accelerator 108, illustrated diagrammatically in Figure 2, includes a tank 201 that comprises an upwardly open container 201a and a closing lid 201b. The lid 201b may be hinged at 202 to the container or tank 201 and includes a locking device on the side diametrically opposite to the hinge. Located in the bottom part of the container is a layer of porous material 203 that includes a cavity 204 which is intended to receive a quantity of freeze-dried microorganisms.

A pipe 205 extends from the lid 201b down into the layer of porous material 203. The pipe 205 terminates beneath the material layer 203 or adjacent the lower limitation of said layer. The upper part of the pipe 205 is connected to a nipple 206 for connection with a source 220 of oxygen-containing gas under pressure, normally air. The source 220 may, for instance, be a pump.

Located above the layer 203 is a plastic or metal net 219 which is adapted to hold the layer of porous material in the lower part of the tank 201. The lid 201b includes a first opening 207 for the delivery of water and/or an aqueous medium that contains microorganisms and for the supply of nutrients, trace substances and other constituents necessary for the growth of microorganisms. The lid 201b also includes a second opening 209 from which a line 221 extends in a direction outwardly of the container or tank 201. The line 221 includes a throttling element, for instance a valve 222, for controlling the pressure in the tank 201. Surplus of the supplied oxygen-containing gas exits through this second opening 209. The tank 201 also includes an outlet port 208 in the lower part of the container, with a connecting line 113 to the collecting tank 115 (Figure 1) for emptying the slurry of microorganisms in the accelerator into the collecting tank 115. The tank 201 may be equipped with heating loops 223 in the container walls, so that the tank contents will be kept at a suitable temperature for optimal micro-organism growth. These heating loops may extend along the full height of the container 201 a, or over parts of said height. The electrical heating loop connections have not been shown.

According to the present invention, a quantity of freeze-dried microorganisms are supplied to the cavity in the porous layer of material 203, whereafter the tank 201 can be closed. Water and/or aqueous medium containing slurried microorganisms and hydrocarbons, trace elements and fertilising substances are then added. Finally, an oxygen containing gas, preferably air, is delivered to the tank 201 so as to keep the aqueous medium in motion and therewith achieve equilibrium in the concentrations of water-dissolved constituents and/or slurried constituents.

In order to produce the high concentration of microorganisms necessary for ground sanitation, it is important that the microorganisms have optimal growth conditions and that optimal conditions for slurrying the freeze-dried microorganisms are achieved. The size of the pores in the porous material is also of great importance. It is also necessary for the pores to connect with one another. The temperature of the medium for increasing the growth of the microorganisms is also important. The temperature should lie within a range of 20-35°C, preferably within a range of 25-30°C. Another important factor is that the fertilising substances can be readily assimilated by the microorganisms; these substances are preferably of organic origin, for instance obtained with the use of algae.

In order to ensure a good yield, there is preferably used water that has been returned from ground sanitation and that contains both microorganisms and hydrocarbons from the sanitised area. These hydrocarbons provide a nutrient for the microorganisms. The water obtained from the sanitised area may be all the water supplied or merely a part of said water. It is, of course, also possible to use pure or clean water.

When the organism reactor lacks the provision of heating loops, the aqueous medium used may be preheated to an optimal temperature for the growth of said microorganisms.

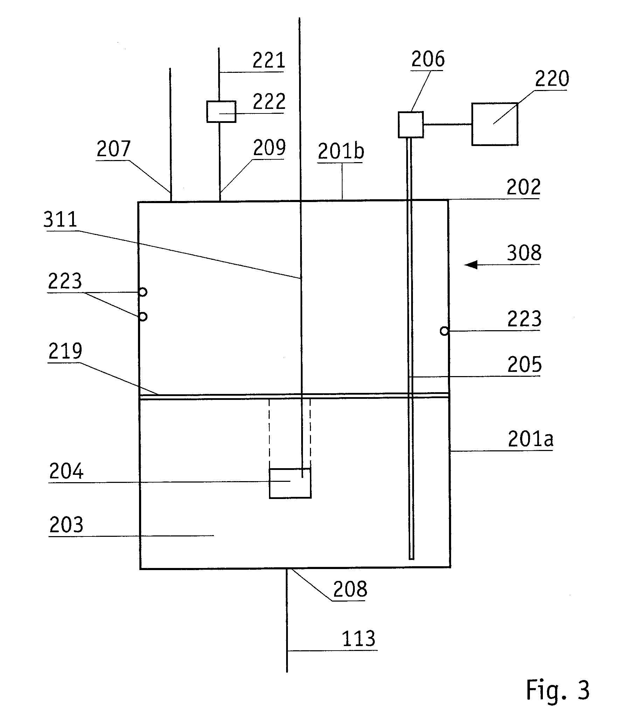

Figure 3 illustrates an organism accelerator 308 for continuous operation, said accelerator 308 corresponding to the organism accelerator 108. Those accelerator components that are also found in the accelerator 108 (Figure 2) have been identified by the same reference signs. The organism accelerator 308 includes a supply pipe 311 for delivering freeze-dried microorganisms to the cavity 204 in the layer 203 of porous material. The delivery pipe 311 is connected to a store of freeze-dried microorganisms and extends from the lid 201 b of the tank 201 down into the layer 203 of said porous material and opens into the cavity 204. A metering device or some corresponding dispenser may be connected to the delivery pipe 311 externally of the tank 201, for metering or dispensing a given quantity of freeze-dried microorganisms.

During operation of the organism accelerator 308, freeze-dried microorganisms are delivered to the tank 201, either continuously or intermittently. Nutrients, trace substances and aqueous medium are also delivered continuously or intermittently, said aqueous medium, or water-containing medium, being either returned from the sanitised area and containing microorganisms and hydrocarbons, or may consist of clean water. The oxygen containing gas is delivered continuously, so as to obtain effective mixing of the slurry. The mean stay time of the aqueous medium ranges from 30 to 60 minutes inclusive. The slurry is removed from the bottom of the tank 1, through the outlet port 208.

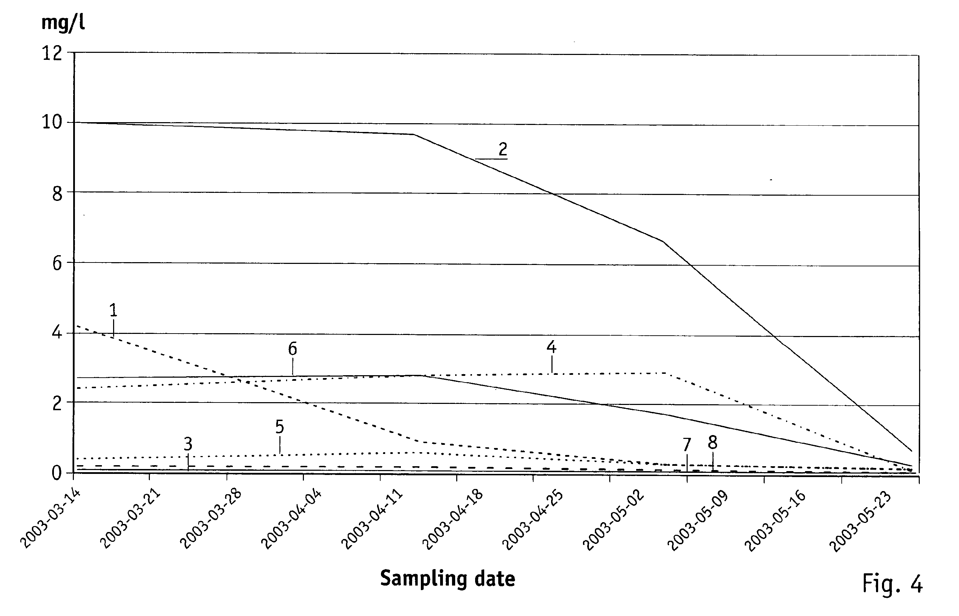

Figure 4 is a graph illustrating the result of a ground sanitation process, in which the concentrations of certain types of hydrocarbons have been plotted in mg/l as a function of time. The curves are referenced 1-8, of which curve 1 refers to aliphatic hydrocarbons, curve 2 relates to aromatic hydrocarbons, curve 3 relates to benzene hydrocarbons, curve 4 relates to toluene, curve 5 relates to ethyl benzene, curve 6 relates to xylene, curve 7 relates to PAH C and curve 8 relates to PAH Ö, where PAH C stands for cancerogenic polyaromatic hydrocarbons and PAH Ö stands for other non-cancerogenic hydrocarbons. It will be seen from the curves that at the beginning of the treatment process, the hydrocarbon concentration slowly falls. After a period of about three months, the hydrocarbon concentration in the sample liquid has fallen to almost zero. One advantage afforded in this respect is that the ground or soil treated is enriched in microorganisms which after sanitation in the absence of nutrients (hydrocarbons) form spores that are activated in response to increasing hydrocarbon concentrations.

## Claims

1. A method of producing a slurry of microorganisms in a liquid medium that consists generally of water, **characterised by** delivering freeze-dried microorganisms to a cavity provided in a material which has mutually communicating pores and which is located in a process container; delivering to said material an aqueous medium, nutrients and trace elements; and causing an oxygen containing gas to flow through the porous material in order to promote the manifold growth of the microorganisms.

2. A method according to Claim 1, **characterised in that** the aqueous medium is contaminated water deriving from or having been obtained from contaminated soil/ground or a contaminated water collection.

3. A method according to Claim 1 or 2, **characterised in that** the aqueous medium contains microorganisms.

4. A method according to Claim 3, **characterised in that** the aqueous medium also contains microorganisms that have been added to the contaminated ground/soil or contaminated water collection.

5. A method according to Claim 3 or 4, **characterised by** subjecting the aqueous medium to conditions for manifolding the growth of microorganisms prior to delivering said aqueous medium to said container, said conditions being achieved by adding nutrients, trace elements and oxygen to said aqueous medium.

6. A method according to one or more of Claims 1-5, **characterised by** keeping the liquid medium at a temperature of 20-35 degrees Celsius.

7. A method according to one or more of Claims 1-6, **characterised in that** the porous material has a porosity of 16-140 pores per cm².

8. A method according to Claim 7, **characterised in that** the porous material has a porosity of 40-100 pores per cm².

9. A method according to Claim 1, **characterised by** delivering the aqueous medium, the freeze-dried microorganisms, the trace elements and the nutrients in a batch-wise process.

10. A method according to Claim 1, **characterised by** delivering the aqueous medium, the freeze-dried microorganisms, the trace elements and the nutrients in a continuous process.

11. An arrangement for carrying out the method according to Claim 10, **characterised in that** the arrangement comprises a tank (201) that includes an inlet opening (207) for the supply of aqueous medium, including nutrients and trace substances for enhancing the growth of microorganisms, and an outlet opening (208) for transportation of the slurry from the tank, a layer (203) of a material that includes mutually communicating pores in the lower part of the container and that also includes a cavity (204) surrounded by porous material and intended for receiving freeze-dried microorganisms, means (205) for delivering oxygen containing gas to the lower part of the container, and a gas outlet opening (209) in the upper part of said container (201b).

12. An arrangement according to Claim 11, **characterised in that** the porous material has a porosity of 16-140 pores per cm².

13. An arrangement according to Claim 12, **characterised in that** the porous material has a porosity of 40-100 pores per cm².

14. An arrangement according to Claim 11, **characterised in that** the porous material has a thickness of 20-70 cm.

15. An arrangement according to Claim 11, **characterised in that** the container is an openable container.

16. An arrangement according to Claim 11, **characterised in that** said arrangement includes a freeze-dried microorganism delivery line (311) through the container enclosure.
